# FASCICULE DE BREVET EUROPEEN

(11) **EP 1 138 315 B1**
(45) Date de publication et mention de la délivrance du brevet: **08.06.2005**
(21) Numéro de dépôt: 01400555.7
(22) Date de dépôt: 02.03.2001
(51) Int. Cl.: A61K 7/06, A45D 34/04

(54) **Applicateur à bille contenant une composition capillaire**
Kugelauftragvorrichtung die ein Haarbehandlungsmittel enthalt
Roll-on applicator containing a hair-treating composition

(30) Priorité: 14.03.2000 FR 0003251
(43) Date de publication de la demande: 04.10.2001
(73) Titulaire: L'OREAL, 75008 Paris (FR)
(72) Inventeur: Jourdan, Hervé, 78210 ST-CYR (FR); Pasquet, Dorothée, 92270 Bois-Colombes (FR)
(74) Mandataire: Bourdeau, Françoise

(56) Documents cités:
- US-A- 3 576 592
- US-A- 5 914 103
- US-A- 5 937 866

## Description

L'invention a pour objet un applicateur à bille (roll-on) contenant une composition capillaire. Elle vise également un procédé cosmétique capillaire comprenant la mise en oeuvre de cet applicateur.

Il est connu des compositions capillaires se présentant sous diverses formes, telles que des crèmes, des lotions, de flacons, des tubes, des bâtons de gels (sticks), des solutions à appliquer à l'aide d'un tampon ou des aérosols

Chaque forme de présentation présente ses inconvénients propres. Par exemple, lorsque la composition se présente sous la forme de crème, de lotion, de flacon ou de tube, l'utilisateur doit prendre la composition entre les doigts et la déposer sur les cheveux. Dans ce cas, il est généralement nécessaire de se laver ou de s'essuyer les mains après l'application du produit capillaire pour éviter de garder des mains collantes, poisseuses ou grasses, ce qui peut rendre incommode l'utilisation de tels produits.

Lorsque la composition se compose sous forme de bâton de gel (stick) ou est appliquée à partir d'un tampon, un inconvénient majeur réside dans le fait que les cheveux restent parfois collés sur le bâton ou le tampon, ce qui pose des problèmes d'hygiène et d'esthétique. La plupart du temps, il est donc déplaisant d'envisager l'utilisation d'un même bâton de gel ou d'un même applicateur à tampon par plusieurs personnes.

Lorsque la composition est appliquée sous forme d'aérosol, elle est distribuée à partir d'un récipient à aérosol par une valve, par l'action d'un agent propulseur. Dans ce cas, le produit diffusé ne peut pas être localisé avec précision mèche à mèche.

On connaît également des applicateurs à bille, par exemple ceux décrit dans les brevets US 3 036 328, US 4 221 494, US 4 221 495, US 4 475 837 ou US 5 051 017. Il a déjà été proposé d'utiliser de tels applicateurs à bille en cosmétique, notamment pour l'application de produits d'hygiène de la peau.

Le document US 5 937 866 divulgue un applicateur à bille pour l' application de composition colorante. Le document WO95/05154 divulgue des compositions nettoyantes pouvant être conditionées dans un applicateur à bille.

Le problème posé par la présente invention est de fournir, pour le maintien de la coiffure et/ou le soin du cheveu, un dispositif renfermant une composition capillaire, qui soit amélioré par rapport aux dispositifs connus dans l'art antérieur, et notamment qui donne lieu à une application commode et hygiénique.

De manière surprenante et inattendue, la Demanderesse a trouvé qu'en sélectionnant, de manière appropriée, les constituants de la composition capillaire d'une part, et certains paramètres d'application de ces compositions par un choix judicieux d'un applicateur à bille, il est possible de résoudre les problèmes énumérés ci-dessus.

L'invention a pour objet un applicateur à bille comprenant un récipient fermé à une extrémité par un moyen de fermeture mobile en rotation, ainsi que des moyens de retenu du moyen de fermeture, le récipient renfermant une composition capillaire de fixation et/ou de maintien de la coiffure exempte de tensioactif carboxylique et comprenant, dans un milieu cosméfiquement acceptable, au moins un composé appartenant aux familles suivantes :
(i) les polymères fixants anioniques, amphotères, non ioniques;
le dispositif étant approprié pour déposer directement sur les cheveux un produit de coiffage ou de soin qui apporte à la coiffure fixation et/ou effet bénéfique sur l'état cosmétique des cheveux et/ou de la brillance.

Un autre objet de la présente invention concerne un procédé cosmétique comprenant la mise en oeuvre d'un tel applicateur à bille.

Le dispositif à bille particulièrement visé par la présente invention est celui commercialisé par la Société Weener Plastic sous l'appellation « ensemble bille et porte bille pour roll'on » (type NARTA).

Le moyen de fermeture peut avoir une forme quelconque, par exemple une forme sphérique, ovale, régulière ou non, ayant une surface lisse ou non.

Au sens de la présente invention, on entend par « effet bénéfique sur l'état cosmétique des cheveux», toute amélioration cosmétique de la qualité des cheveux ou de leur apparence, notamment l'amélioration de la facilité de démêlage, de la douceur au toucher ou du lissage des cheveux ou encore de leur aspect visuel, comme leur coloration.

Par le mot « composition capillaire », on entend une composition pour le maintien et/ou la fixation de la coiffure, le soin des cheveux, le maquillage des cheveux ou la coloration des cheveux.

Par le mot « coiffage », on entend le fait de fixer et/ou de maintenir la forme de la coiffure. Par le mot « soin », on entend le fait d'améliorer ou de maintenir les propriétés cosmétiques ou mécaniques naturelles des cheveux.

Au sens de la présente invention, on entend par « polymère fixant », un polymère capable de fixer et/ou de maintenir la forme de la coiffure.

Les polymères fixants peuvent être utilisés sous forme solubilisé ou sous forme de dispersion de particules solides de polymères.

Les polymères fixants anioniques, amphotères et non ioniques utilisables conformément à l'invention sont décrits ci-après.

Les polymères fixants anioniques généralement utilisés sont des polymères comportant des groupements dérivés d'acide carboxylique, sulfonique ou phosphorique et ont un poids moléculaire compris entre environ 500 et 5.000.000.

### 1) Les groupements carboxyliques sont apportés par des monomères mono ou diacides carboxyliques insaturés tels que ceux répondant à la formule :

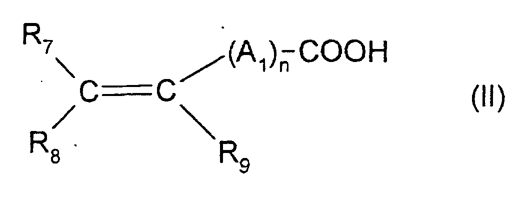

dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées
   ou monoamidifiées.
   Ces polymères sont par exemple décrits dans les brevets français 2 350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.
Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale III: 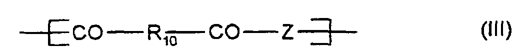 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono
   ou bis-secondaire et de préférence représente :
   a) dans les proportions de 60 à 100 moles %, le radical IV 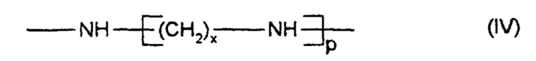 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 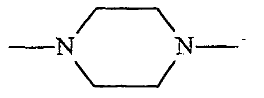
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule V: 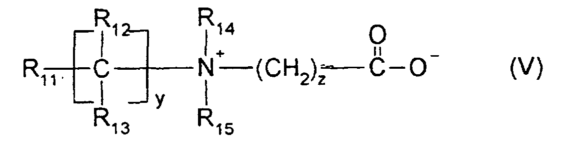 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 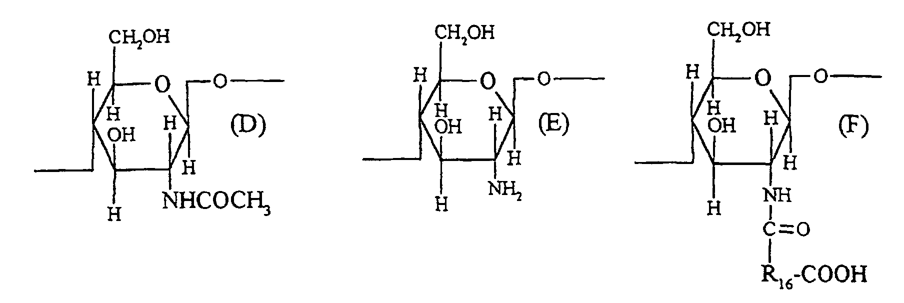 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 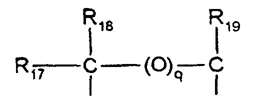 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366 : 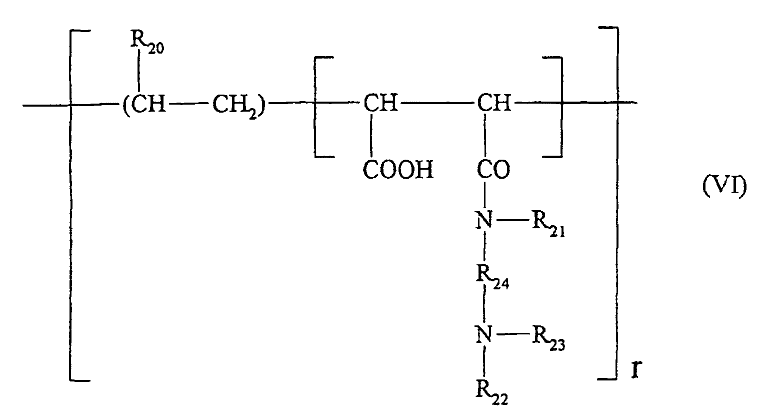 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 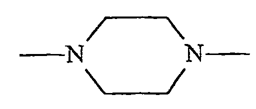 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII)'
   où D désigne un radical 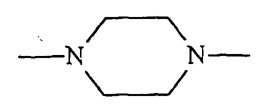 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213
ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées.

Les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C₁-C ₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Les polymère non ionique qui conviennent tout particulièrement pour la réalisation des compositions conformes à l'invention sont ceux choisis parmi:
* les copolymères de vinyllactame tels que les copolymères de vinylpyrrolidone et d'acétate de vinyle et les copolymères vinylpyrrolidone/ acétate de vinyle/ propionate de vinyle
* la polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle,
b) 0 à 40% en poids d'acide acrylique ;
c) 5 à 40% en poids de macromère siliconé de formule : 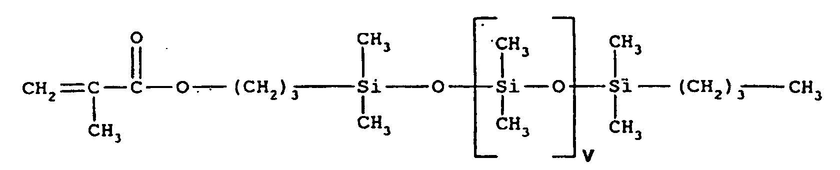 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non.

Selon l'invention, le ou les polymères fixants peuvent représenter de 0,1 % à 10 % en poids, de préférence de 0,5 % à 5% en poids par rapport au poids total de la composition finale.

Le milieu cosmétiquement acceptable est, de préférence, constitué par de l'eau ou un ou plusieurs solvants cosmétiquement acceptables tels que des alcools ou les silicones volatiles cycliques ou des mélanges eau-solvant(s), ces solvants étant de préférence des alcools en C₁-C₄.

La concentration en solvant non aqueux peut varier de 0 à 99,9 %, et de préférence de 0 à 60 % du poids de la composition.

La composition selon l'invention peut également contenir un agent épaississant, de préférence polymérique.

Au sens de la présente invention, on entend par agent épaississant, tout agent capable d'augmenter la viscosité d'une composition cosmétique.

Les épaississants pouvant être utilisés conformément à l'invention peuvent être d'origine naturelle ou synthétique. On peut citer comme épaississant-naturels convenant pour l'invention les gommes de xanthane, de scléroglucane, de gellane, de rhamsan, les alginates, la maltodextrine, l'amidon et ses dérivés, la gomme de karaya, la farine de caroube, les gommes de guar, les celluloses et ses dérivés.

Les agents épaississants synthétiques selon l'invention sont avantageusement des polymères ou copolymères d'acide acrylique et/ou méthacrylique, comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate commercialisé par la société GUARDIAN sous la dénomination Lubragel ou encore le polyglycérylacrylate commercialisé sous la dénomination Hispagel par la société HISPANO CHIMICA.

Comme autres composés acryliques, on peut citer les copolymères d'acide acrylique ou méthacrylique comprenant au moins un motif acrylate d'alkyle en C1 à C30 et/ou un motif uréthanne éventuellement substitué par une chaîne grasse. On peut en particulier citer le Pemulen TR1 (Goodrich), le Viscophobe DB 1000 (Union Carbide) et les Acrysol 44 ou 46 (Rohm et Haas).

On peut encore utiliser comme agent épaississant les polyéthylèneglycols (PEG) et leurs dérivés.

On peut également utiliser avantageusement, en tant qu'agent épaissisant, des polyacrylamides épaississants. Ceux-ci peuvent plus particulièrement être choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium éventuellement réticulés,
- les copolymères d'acrylamide et d'acide 2-acrylamido 2-méthyl propanesulfonique partiellement ou totalement neutralisés éventuellement réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau.

La composition de l'invention peut également contenir au moins un additif choisi parmi les tensioactifs anioniques, non ioniques ou amphotères, les polyols comme le glycol ou la glycérine, les pigments, les colorants, les parfums, les filtres, les conservateurs, les protéines, les vitamines, les provitamines, les polymères autres que ceux de l'invention et tout autre additif classiquement utilisé dans les compositions cosmétiques.

Les applicateurs à bille selon l'invention peuvent être utilisés pour déposer sur les cheveux notamment, des compositions de soin, des compositions de fixation et/ou de maintien de la coiffure, des après-shampooings, des baumes pour les cheveux, des conditionneurs.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

L'invention va être plus complètement illustrée à l'aide des exemples suivants.

Tous les pourcentages sont des pourcentages relatifs en poids par rapport au poids total de la composition et m.a. signifie matière active.

Les constituants utilisés sont rassemblés dans le tableau ci-après.

| | |
|---|---|
| Synthalen K | Polymère acrylique commercialisé par 3 V |
| Mirasil DMCO | Diméthicone copolyol commercialisée par Rhône Poulenc |
| Jaguar HP 105 | Gomme hydroxypropyl commercialisée par Rhône Poulenc |
| Arquad 16-25 LO | Chlorure de cétrimonium commercialisé par Akzo Nobel |
| Gafquat 734 | Polyquaternium 11 commercialisé par ISP |
| Celquat LOR | Polyquaternium 4 commercialisé par National Starch |
| Solanace Starch | Amidon de pomme de terre modifié commercialisé par National Starch |
| Ultrez 10 | Carbomer commercialisé par Goodrich |
| Luvimer MAE | Copolymère acrylate commercialisé par BASF |
| DC x et DCx fluid | Silicone commercialisée par Dow Corning |
| Genamin | Chlorure de behenalkomium commercialisé par Hoechst |
| Aristoflex A | Copolymère acétate de vinyle/acide crotonique/polyéthylèneglycol en solution à 60 % dans un mélange IPA 87,4/eau 12,6 |
| Polyéthylène glycol 400 | Polymère d'oxyde d'éthylène commercialisé par Dow Chemical |
| Abil B8842 | Céthyldiméthicone copolyol commercialisé par Goldschmidt |
| Aculyn 22 | Copolymère acrylate/méthacrylate commercialisé par Rohm & Haas |
| Ultrez 10 | Polymère acrylique commercialisé par Goodrich |
| Jaguar HP 105 | Gomme commercialisée par Rhone Poulenc |
| Ultrahold Strong | Copolymère acrylate commercialisé par BASF |

### Exemples

On fabrique des applicateurs à bille selon l'invention contenant diverses compositions cosmétiques A à H faisant l'objet des exemples 1 à 6 ci-après.

### Exemple 1

| | **A** | **B** |
|---|---|---|
| Synthalen K | 0.5 | 1 |
| Aristoflex A | 3% m.a. | |
| Mirasil DMCO | 0.1 | |
| Parfum | 0.3 | |
| Ethanol | 40 | |
| AMP | qsp pH 7.6 | |
| Eau | qsp 100 | |

### Exemple 2

| | **C** |
|---|---|
| Jaguar HP 105 | 0.5 |
| Glycérol | 0.5 |
| Arquad 16-25 LO | 2 |
| Mirasil DMCO | 0.5 |
| PVP | 2 |
| Gafquat 734 | 0.2 |
| Celquat LOR | 0.2 |
| Parfum | 0.15 |
| Ethanol | 35 |
| AMP | qsp pH 7.2 |
| Eau | qsp 100 |

### Exemple 3

| | **D** |
|---|---|
| Solanace Starch | 0.25 |
| Ultrez 10 | 0.4 |
| Luvimer MAE | 6.6 |
| Celquat LOR | 0.3 |
| DC 2-1182 | 3 |
| Genamin | 0.45 |
| DC 939 | 0.5 |
| Propylène glycol | 2.5 |
| Conservateurs | 0.7 |
| Parfum | 0.4 |
| AMP | qsp pH 7.5 |
| Eau | qsp 100 - |

### Exemple 4

| | **E** | **F** |
|---|---|---|
| DC 556 fluid | 15 | |
| DC 200 fluid | 25 | 30 |
| Parfum | 0.15 | |
| Ethanol | 5 | |
| DC 245 fluid | qsp 100 | |

### Exemple 5

| | **G** |
|---|---|
| Aristoflex A | 5 (3 % m.a) |
| Glycérol | 11 |
| Polyéthylèneglycol 400 | 2.2 |
| Abil B8842 | 1.7 |
| Aculyn 22 | 1.7 |
| Parfum | 0.2 |
| Conservateurs | 0.7 |
| Triéthanomamine | qsp pH 8 |
| Eau | qsp 100 |

### Exemple 6

| | **H** |
|---|---|
| Ultrez 10 | 0.3 |
| Luvimer MAE | 6.6 |
| Jaguar HP105 | 0.05 |
| Ultrahold Strong | 0.5 |
| DC 556 fluid | 20 |
| DC 245 fluid | 22 |
| AMP | qsp pH 7.5 |
| Eau | qsp 100 |

Les compositions A à H conformes à l'invention, faisant l'objet des exemples 1 à 6, sont introduites dans le dispositif à bille commercialisé par Weener Plastic sous l'appellation « Ensemble bille et porte bille roll'on ». On applique les compositions sur les cheveux. En application de finition, l'application directe permet d'agir sur l'ensemble de la chevelure, et préférentiellement de procéder à des retouches de façon localisée.

On obtient, selon le type de produit utilisé, des coiffures dont les mèches :
- sont maintenues avec une fixation de normale à extra forte,
- sont mieux maîtrisées avec plus de corps et de coiffant,
- donnent l'impression d'être légèrement humide ( un effet « wet »),
- ont plus de douceur et de facilité de démêlage,
   - ont une brillance accentuée.

## Revendications

1. Applicateur à bille comprenant un récipient fermé à une extrémité par un moyen de fermeture mobile en rotation, ainsi que des moyens de retenu du moyen d fermeture, le récipient renfermant une composition capillaire de fixation et/ou de maintien de la coiffure exempte de tensioactif carboxylique et comprenant, dans un milieu cosmétiquement acceptable, au moins un composé appartenant aux familles suivantes :
(i) les polymères fixants anioniques, amphotères, non ioniques;
l'applicateur étant approprié pour déposer directement sur les cheveux un produit de coiffage ou de soin qui apporte à la coiffure fixation et/ou effet bénéfique sur l'état cosmétique des cheveux et/ou de la brillance.

2. Applicateur selon la revendication 1, **caractérisé par le fait que** le polymère fixant est un polymère anionique choisi parmi :
- les polymères comportant des motifs carboxyliques dérivant de monomères mono ou diacides carboxyliques insaturés de formule : 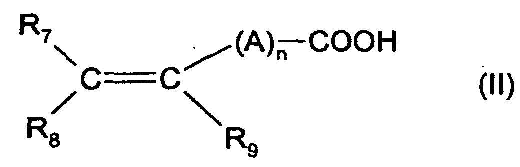 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle; R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

3. Applicateur selon la revendication 1, **caractérisé en ce que** le polymère fixant est un polymère amphotère choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

4. Applicateur selon la revendication 1, **caractérisé par le fait que** le polymère fixant est un polymère non ionique choisi parmi :
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle;
- les copolymères d'acrylonitrile et d'un monomère non ionique choisi par exemple parmi le butadiène et les (méth)acrylates d'alkyle;
- les copolymères d'acrylate d'alkyle et d'uréthanne ; et
- les polyamides ; et
- les gommes de guar non ioniques chimiquement modifiées ou non modifiées.

5. Applicateur selon la revendication 1, **caractérisé par le fait que** le polymère fixant est un polyuréthanne fonctionnalisé ou non, siliconé ou non.

6. Applicateur selon la revendication 1, **caractérisé par le fait que** le polymère fixant est un polymère de type siliconé greffé comprenant une portion polysiloxane et une portion constituée d'une chaîne organique non-siliconée, l'une des deux portions constituant la chaîne principale du polymère l'autre étant greffée sur la dite chaîne principale.

7. Applicateur selon l'une quelconque des revendications précédentes, **caractérisé en ce que** le ou les polymères fixants peuvent représenter de 0,1 % à 10 % en poids, de préférence de 0,5 % à 5% en poids par rapport au poids total de la composition finale.

8. Applicateur selon l'une quelconque des revendications précédentes, **caractérisé par le fait que** la composition contient, en outre, au moins un additif choisi parmi les tensioactifs anioniques, non ioniques ou amphotères, les polyols comme le glycol ou la glycérine, les pigments, les colorants, les parfums, les filtres, les conservateurs, les protéines, les vitamines, les provitamines, les polymères autres que ceux de l'invention et tout autre additif classiquement utilisé dans les compositions cosmétiques.

9. Procédé cosmétique, **caractérisé par le fait qu'**il comprend la mise en oeuvre d'un applicateur selon l'une quelconque des revendications précédentes.

## Patentansprüche

1. Kugelapplikator, der einen Behälter, welcher an einem Ende mithilfe eines drehbaren Verschlussmittels verschlossen ist, und Halteeinrichtungen für das Verschlussmittel aufweist, wobei der Behälter eine Zusammensetzung für die Haarbehandlung zur Formgebung und/oder Festigung der Frisur enthält, die keinen grenzflächenaktiven Stoff mit Carboxygruppe enthält und in einem kosmetisch akzeptablen Medium mindestens eine Verbindung enthält, die aus den folgenden Gruppen stammt:
(i) anionischen, amphoteren, nichtionischen fixierenden Polymeren;
wobei die Vorrichtung geeignet ist, um ein Produkt zur Frisurengestaltung oder zur Pflege, das die Frisur fixiert und/oder eine günstige Wirkung auf den kosmetischen Zustand und/oder den Glanz hat, direkt auf die Haare aufzubringen.

2. Applikator nach Anspruch 1, **dadurch gekennzeichnet, dass** das fixierende Polymer ein anionisches Polymer ist, das ausgewählt ist unter:
- Polymeren, die Carboxyeinheiten aufweisen, die von ungesättigten Mono- oder Dicarbonsäuremonomeren der folgenden Formel abgeleitet sind: 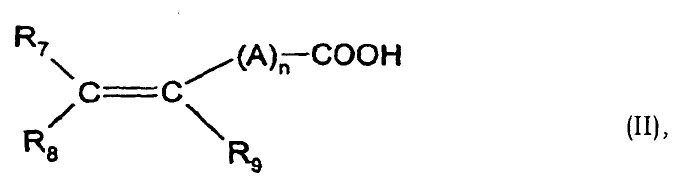 worin bedeuten: n 0 oder eine ganze Zahl von 1 bis 10, A eine Methylengruppe, die gegebenenfalls über ein Heteroatom, wie Sauerstoff oder Schwefel, an das Kohlenstoffatom der ungesättigten Gruppe oder, wenn n größer 1 ist, an die benachbarte Methylengruppe gebunden ist, R₇ ein Wasserstoffatom, eine Phenylgruppe oder eine Benzylgruppe, R₈ ein Wasserstoffatom, eine niedere Alkylgruppe oder eine Carboxygruppe und R₉ ein Wasserstoffatom, eine niedere Alkylgruppe, die Gruppe -CH₂-COOH, eine Phenylgruppe oder eine Benzylgruppe;
- Polymeren, die Einheiten enthalten, die von Sulfonsäure abgeleitet sind, wie beispielsweise Vinylsulfon-, Styrolsulfon-, Acrylamidoalkylsulfoneinheiten.

3. Applikator nach Anspruch 1, **dadurch gekennzeichnet, dass** das fixierende Polymer ein amphoteres Polymer ist, das unter den Polymeren ausgewählt ist, die Einheiten enthalten, die abgeleitet sind von:
a) mindestens einem Monomer, das unter den Acrylamiden oder Methacrylamiden ausgewählt ist, die am Stickstoffatom mit einer Alkylgruppe substituiert sind,
b) mindestens einem sauren Comonomer, das eine oder mehrere reaktive Carboxygruppen enthält, und
c) mindestens einem basischen Comonomer, beispielsweise Acrylsäureestern und Methacrylsäureestern mit primären, sekundären, tertiären und quartären Aminosubstituenten und dem Produkt der Quaternisierung von Dimethylaminoethylmethacrylat mit Dimethylsulfat oder Diethylsulfat.

4. Applikator nach Anspruch 1, **dadurch gekennzeichnet, dass** das fixierende Polymer ein nichtionisches Polymer ist, das ausgewählt ist unter:
- Polyalkyloxazolinen;
- Vinylacetat-Homopolymeren;
- Vinylacetat/Acrylester-Copolymeren;
- Vinylacetat/Ethylen-Copolymeren;
- Vinalacetat/Maleinsäureester-Copolymeren;
- Polyethylen/ Maleinsäureanhydrid-Copolymeren;
- Homopolymeren von Alkylacrylaten und Homopolymeren von Alkylmethacrylaten;
- Acrylester-Copolymeren, beispielsweise den Copolymeren von Alkylacrylaten und Alkylmethacrylaten;
- Copolymeren von Acrylnitril und einem nichtionischen Monomer, das beispielsweise unter Butadien und den Alkyl(meth)acrylaten ausgewählt ist;
- Alkylacrylat/Urethan-Copolymeren;
- Polyamiden; und
- chemisch modifizierten oder nicht modifizierten nichtionischen Guargummen.

5. Applikator nach Anspruch 1, **dadurch gekennzeichnet, dass** das fixierende Polymer ein funktionalisiertes oder nicht funktionalisiertes, siliconhaltiges oder nicht siliconhaltiges Polyurethan ist.

6. Applikator nach Anspruch 1, **dadurch gekennzeichnet, dass** das fixierende Polymer ein Polymer vom gepfropften Silicontyp ist, das einen Polysiloxanbereich und einen Bereich aufweist, der von einer nicht siliconhaltigen organischen Kette gebildet wird, wobei ein Bereich die Hauptkette des Polymers bildet und der andere Bereich auf die Hauptkette gepfropft ist.

7. Applikator nach einem der vorhergehenden Ansprüche, **dadurch gekennzeichnet, dass** das fixierende Polymer oder die fixierenden Polymere 0,1 bis 10 Gew.-% und vorzugsweise 0,5 bis 5 Gew.-%, bezogen auf das Gesamtgewicht der fertigen Zusammensetzung, ausmachen können.

8. Applikator nach einem der vorhergehenden Ansprüche, **dadurch gekennzeichnet, dass** die Zusammensetzung ferner mindestens einen Zusatzstoff enthält, der unter den anionischen, nichtionischen oder amphoteren grenzflächenaktiven Stoffen, Polyolen wie Glykol oder Glycerin, Pigmenten, Farbmitteln, Parfums, Filtern, Konservierungsmitteln, Proteinen, Vitaminen, Provitaminen, Polymeren, die von den erfindungsgemäßen Polymeren verschieden sind, und beliebigen anderen, herkömmlich in kosmetischen Zusammensetzungen verwendeten Zusatzstoffen ausgewählt ist.

9. Kosmetisches Verfahren, **dadurch gekennzeichnet, dass** es die Verwendung eines Applikators nach einem der vorhergehenden Ansprüche umfasst.

## Claims

1. Roll-on applicator comprising a container closed at one end by a closure means which can move in rotation and means for retaining the closure means, the container including a hair composition for fixing and/or retaining the form of the hairstyle devoid of carboxylic surfactant which comprises, in a cosmetically acceptable medium, at least one compound belonging to
(i) anionic, amphoteric or nonionic fixing polymers;
the applicator being appropriate for directly depositing on the hair a styling or care product which contributes, to the hairstyle, fixing and/or a beneficial effect on the cosmetic condition of the hair and/or sheen.

2. Applicator according to Claim 1, **characterized in that** the fixing polymer is an anionic polymer chosen from:
- polymers comprising carboxyl units deriving from unsaturated mono- or dicarboxylic acid monomers of formula: 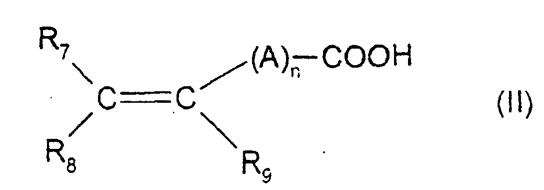 in which n is an integer from 0 to 10, A denotes a methylene group, optionally connected to the carbon atom of the unsaturated group or to the neighbouring methylene group when n is greater than 1 via a heteroatom, such as oxygen or sulphur, R₇ denotes a hydrogen atom or a phenyl or benzyl group, R₈ denotes a hydrogen atom or a lower alkyl or carboxyl group, and R₉ denotes a hydrogen atom, a lower alkyl group or a -CH₂-COOH, phenyl or benzyl group;
- polymers comprising units deriving from sulphonic acid, such as vinylsulphonic, styrenesulphonic or acrylamidoalkylsulphonic units.

3. Applicator according to Claim 1, **characterized in that** the fixing polymer is an amphoteric polymer chosen from polymers comprising units deriving:
a) from at least one monomer chosen from acrylamides or methacrylamides substituted on the nitrogen by an alkyl radical,
b) from at least one acidic comonomer comprising one or more reactive carboxyl groups, and
c) from at least one basic comonomer, such as esters comprising primary, secondary, tertiary and quaternary amine substituents of acrylic and methacrylic acids and the quaternization product of dimethylaminoethyl methacrylate with dimethyl or diethyl sulphate.

4. Applicator according to Claim 1, **characterized in that** the fixing polymer is a nonionic polymer chosen from:
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- copolymers of polyethylene and of maleic anhydride;
- alkyl acrylate homopolymers and alkyl methacrylate homopolymers;
- acrylic ester copolymers, such as, for example, copolymers of alkyl acrylates and of alkyl methacrylates;
- copolymers of acrylonitrile and of a nonionic monomer chosen, for example, from butadiene and alkyl (meth)acrylates;
- copolymers of alkyl acrylate and of urethane;
- polyamides; and
- chemically modified or unmodified nonionic guar gums.

5. Applicator according to Claim 1, **characterized in that** the fixing polymer is a functionalized or nonfunctionalized polyurethane which may or may not comprise silicones.

6. Applicator according to Claim 1, **characterized in that** the fixing polymer is a polymer of grafted silicone type comprising a polysiloxane portion and a portion composed of a non-silicone organic chain, one of the two portions constituting the main chain of the polymer and the other being grafted onto the said main chain.

7. Applicator according to any one of the preceding claims, **characterized in that** the fixing polymer or polymers can represent from 0.1% to 10% by weight, preferably from 0.5% to 5% by weight, with respect to the total weight of the final composition.

8. Applicator according to any one of the preceding claims, **characterized in that** the composition additionally comprises at least one additive chosen from anionic, nonionic or amphoteric surfactants, polyols, such as glycol or glycerol, pigments, dyes, fragrances, screening agents, preservatives, proteins, vitamins, provitamins, polymers other than those of the invention and any other additive conventionally used in cosmetic compositions.

9. Cosmetic process, **characterized in that** it comprises the use of an applicator according to any one of the preceding claims.
